(19) Europäisches Patentamt
European Patent Office
Office européen des brevets

(11) Veröffentlichungsnummer: **0 141 328**
**B1**

(12) **EUROPÄISCHE PATENTSCHRIFT**

(45) Veröffentlichungstag der Patentschrift: **18.04.90**

(21) Anmeldenummer: **84112374.8**

(22) Anmeldetag: **13.10.84**

(51) Int. Cl.$^5$: **C 07 C 217/04,**
C 07 C 217/48, C 07 C 213/00,
C 07 C 213/04

# (54) Verfahren zur Herstellung tertiärer Amine.

(30) Priorität: **22.10.83 DE 3338432**

(43) Veröffentlichungstag der Anmeldung:
**15.05.85 Patentblatt 85/20**

(45) Bekanntmachung des Hinweises auf die Patenterteilung:
**18.04.90 Patentblatt 90/16**

(84) Benannte Vertragsstaaten:
**DE**

(56) Entgegenhaltungen:
**DE-A-2 642 517**
**GB-A-2 059 792**

**CHEMICAL ABSTRACTS, Band 76, Nr. 18, 1. Mai 1972, Columbus, Ohio, US; Seite 44, Spalte 1, Abstrakt Nr. 100 665 h; & JP - B - 71 01857 (SUMITOMO CHEMICAL) 18-01-1971 (Kat. A,D)**

(73) Patentinhaber: **HOECHST AKTIENGESELLSCHAFT**
**Postfach 80 03 20**
**D-6230 Frankfurt am Main 80 (DE)**

(72) Erfinder: **Wester, Norbert, Dr.**
**Sachsenring 12**
**D-6238 Hofheim am Taunus (DE)**

**EP 0 141 328 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der Erfindung ist die Herstellung von tertiären Aminen der allgemeinen Formel

$$R^1(-OCH_2CH_2)_n$$
\
N—$R^2$
/
$$R^1(-OCH_2CH_2)_n$$

worin $R^1=C_8-C_{26}$-Alkyl, $C_9-C_{18}$-Alkyl-aryl, $R^2=C_1-C_{22}$-Alkyl und n=1—15 bedeuten.

Verbindungen dieses Typs können durch Aminolyse aus den entsprechenden Alkoholen, Aldehyden oder Ketonen und primären Alkylaminen (DE 30 34 433) dargestellt werden. Eine weitere Synthesemöglichkeit besteht in der Alkylierung von ebenfalls durch Aminolyse zugänglichen sekundären Etheraminen (US 2 285 419, US 3 373 204, US 2 928 877). Die Umsetzungen nach diesen Verfahren verlaufen jedoch nicht einheitlich. Man erhält Substanzgemische primärer, sekundärer und tertiärer Amine, die selbst bei sehr kleinen Resten $R^1$, $R^2$ und niedrigen Werten für n nur noch mit recht aufwendigen Methoden getrennt werden können. Außerdem tritt durch Spaltreaktionen eine Reihe unerwünschter Nebenprodukte wie umweltbelastender, niedrig siedender Amine auf. Eine technische Durchführung dieser Verfahren ist daher insbesondere für Verbindungen mit größeren Resten $R^1$, $R^2$, wenn überhaupt, nur unter großen Schwierigkeiten möglich und dürfte in den meisten Fällen unwirtschaftlich sein.

Bakannt ist auch die Umsetzung von Octadecylamin mit 2 Mol 2 - Methyl - ß - chlorethylether (JA 71 01 857). Diese Reaktion arbeitet jedoch mit einer nur schwach basischen Alkaliverbindung ($K_2CO_3$) und die Reaktionszeit beträgt 30 bis 50 Stunden.

Es wurde nun gefunden, daß sich die gewünschten tertiären Amine gezielt und in hohen Ausbeuten durch Alkylierung von primären Alkylaminen herstellen lassen, wenn man die Reaktion bei relativ hohen Temperaturen und in Gegenwart von starken Basen durchführt.

Gegenstand der Erfindung ist somit ein Verfahren zur Herstellung von tertiären Aminen der Formel

$$R^1-(OCH_2CH_2)_n$$
\
N—$R^2$
/
$$R^1-(OCH_2CH_2)_n$$

worin $R^1=C_8-C_{26}$-Alkyl oder $C_9-C_{18}$-Alkyl-aryl, $R^2=C_1-C_{22}$-Alkyl und n eine Zahl von 1—15 bedeuten, das dadurch gekennzeichnet ist, daß man 2 Mol einer Verbindung der Formel

$$R^1-(OCH_2CH_2)_n-X$$

worin X Halogen, vorzugsweise Chlor, oder $-OSO_3Na$ oder $-OSO_3K$ bedeutet und $R^1$ und n die oben angegebenen Bedeutungen haben, mit 1 bis 1,4 Mol einer Verbindung der Formel

$$R^2-NH_2$$

wobei $R^2$ ein $C_1-C_{22}$-Alkyl-Rest bedeutet, in Gegenwart einer mindestens äquimolaren Menge einer starken Base bei Temperaturen von 100 bis 200°C, vorzugsweise 160 bis 185°C, in einem Lösemittel oder Lösemittelgemisch umsetzt.

Die Reaktion wird bevorzugt in Wasser als Lösemittel durchgeführt. Als starke Base kommen bevorzugt Kaliumhydroxid und Natriumhydroxid in Frage. Diese Basen werden eingesetzt in mindestens äquimolaren Mengen, bezogen auf die alkylierende Verbindung. Bevorzugt ist ein Überschuß an Base in einer Menge von 10 bis 30%. Die Reaktionszeit beträgt im allgemeinen 3 bis 10 Stunden. Die Endprodukte können problemlos bei Temperaturen oberhalb ihrer Trübungspunkte, eventuell unter Zusatz von Salzen, aus der wäßrigen Lösung oder Emulsion isoliert werden. Die erfindungsgemäß erhaltenen tertiären Amine dienen als Ausgangsverbindungen zur Herstellung der entsprechenden quartären Ammoniumverbindungen.

Bei dem erfindungsgemäßen Verfahren ist es als überraschend anzusehen, daß in der stark alkalischen Lösung und bei den hohen Reaktionstemperaturen die an sich zu erwartende Verseifung der Ausgangshalogenide bzw. -sulfate nur in untergeordnetem Ausmaß auftritt. Dies ist umso erstaunlicher, als die reaktiven Zentren der Ausgangsverbindungen durch die Oxethylen-Einheiten wasserlöslich werden und damit für den Angriff der Hydroxyl-Ionen leicht zugänglich sind. Ein weiteres überraschendes Merkmal dieses Verfahrens ist die Selektivität der Reaktion. Sie führt in Ausbeuten von über 85% direkt zu den tertiären Aminen. Obwohl kein Überschuß an Halogenid bzw. Sulfat eingesetzt wird, enthält das Endprodukt wider Erwarten, wenn überhaupt, nur ganz geringe Mengen des entsprechenden, als Zwischenprodukt gebildeten sekundären Amins. Die folgenden Beispiele sollen die Möglichkeiten des erfindungsgemäßen Verfahrens verdeutlichen.

Beispiel 1

Bis(cocosalkylpentaoxethyl)methylamin

460 g (1,0 Mol) Cocosalkylpentaoxethylchlorid, 38,8 g (0,5 Mol) 40%ige Methylamin-Lsg. 48 g (1,2 Mol) NaOH und 450 ml entionisiertes Wasser (E-Wasser) werden in einen 2 l Druckautoklaven gefüllt und 10 h bei 175°C gerührt. Man trennt die organische Schicht ab und entfernt das in ihr verbliebene Wasser durch Destillation im Vakuum. Man erhält 420 g (95,7% d.Th.) tertiäres Amin.

tert N: gef. 1,5% ber. 1,6%

Beispiel 2

Bis(isotridecylpentaoxethyl)octylamin

140 g (0,32 Mol) Isotridecylpentaoxethylchlorid, 20,6 g (0,16 Mol) n-Octylamin, 15,4 (0,38 Mol NaOH und 600 ml E-Wasser werden in einen Druckautoklaven gefüllt und 10 h bei 175°C gerührt. Die Aufarbeitung erfolgt entsprechend Beispiel 1.

Ausbeute: 133 g (88% d.Th.)

tert N: gef. 1,4 ber. 1,5

Beispiel 3

Bis(nonylphenylhexaoxethyl)methylamin

482 g (1,0 Mol) Nonylphenylhexaoxethylchlorid, 40 g (0,52 Mol) Methylamin-Lsg. (40%), 48 g NaOH und 500 ml Isopropanol werden 10 h bei 175°C in einem Autoklaven gerührt. Es wird vom Salz abgesaugt und das Lösungsmittel im Vakuum abdestilliert.

Ausbeute: 437 g (94,8% d.Th.)

tert N: gef. 1,4 ber. 1,5

Beispiel 4

Bis($C_{12/14}$alkyltrioxethyl)methylamin

750 g (1,18 Mol) $C_{12/14}$-alkyltrioxethylsulfat-Natrium (70%) 45,7 g (0,59 Mol) Methylamin-Lsg. (40%), 48 g (1,2 Mol) NaOH und 400 ml E-Wasser werden entsprechend Beispiel 1 umgesetzt und aufgearbeitet. Ausbeute 366 g (94,1% d.Th.)

tert N: gef. 1,95 ber. 2,1

Beispiel 5

Bis($C_{20/22}$alkyldioxethyl)methylamin

390 g (1,0 Mol $C_{20/22}$alkyldioxethylchlorid, 46,5 g (0,6 Mol) Methylamin-Lsg. (40%), 48 g (1,2 Mol) NaOH und 600 ml E-Wasser werden entsprechend Beispiel 1 umgesetzt und aufgearbeit, jedoch bei einer Reaktionszeit von 5 Stunden.

Ausbeute: 342 g (92,7% d.Th.)

tert N: gef. 1,6 ber. 1,75

**Patentanspruch**

Verfahren zur Herstellung von tertiären Aminen der Formel

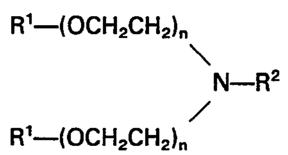

worin $R^1$ $C_8$—$C_{22}$-Alkyl oder $C_9$—$C_{18}$-Alkyl-aryl, $R^2$ $C_1$—$C_{22}$-Alkyl und n eine Zahl von 1—15 bedeuten, dadurch gekennzeichnet, daß man 2 Mol einer Verbindung der Formel

$$R^1\text{—}(OCH_2CH_2)_n\text{—}X$$

worin X Halogen, —$OSO_3Na$ oder —$OSO_3K$ bedeutet und $R^1$ und n die oben angegebenen Bedeutungen haben, mit 1 bis 1,4 Mol einer Verbindung der Formel

$$R^2\text{—}NH_2$$

wobei $R^2$ ein $C_1$—$C_{22}$-Alkyl-Rest bedeutet, in Gegenwart einer mindestens äquimolaren Menge einer starken Base bei Temperaturen von 100 bis 200°C, vorzugsweise 160 bis 185°C, in einem Lösemittel oder Lösemittelgemisch, vorzugsweise in Wasser, umsetzt.

**Revendication**

Procédé pour préparer des amines tertiaires répondant à la formule:

$$\begin{array}{l} R^1—(OCH_2CH_2)_n \\ \qquad\qquad\qquad\quad \diagdown \\ \qquad\qquad\qquad\qquad N—R^2 \\ \qquad\qquad\qquad\quad \diagup \\ R^1—(OCH_2CH_2)_n \end{array}$$

dans laquelle $R^1$ représente un alkyle en $C_8—C_{26}$ ou un $C_9—C_{18}$-alkyl-aryle, $R^2$ représente un alkyle en $C_1—C_{22}$ et n désigne un nombre de 1 à 15, procédé caractérisé en ce qu'on fait réagir 2 mol d'un composé de formule:

$$R^1—(OCH_2CH_2)_n—X$$

dans laquelle X représente un halogène, $—OSO_3Na$ ou $—OSO_3K$, et $R^1$ et n ont les significations qui leur ont été données ci-dessus, avec de 1 à 1,4 mol d'un composé de formule:

$$R^2—NH_2$$

dans laquelle $R^2$ représente un alkyle en $C_1—C_{22}$, en présence d'une quantité au moins équimolaire d'une base forte, à des températures de 100 à 200°C, de préférence de 160 à 185°C, dans un solvant ou un mélange de solvants, de préférence dans de l'eau.

**Claim**

Process for preparing tertiary amines of the formula

$$\begin{array}{l} R^1—(OCH_2CH_2)_n \\ \qquad\qquad\qquad\quad \diagdown \\ \qquad\qquad\qquad\qquad N—R^2 \\ \qquad\qquad\qquad\quad \diagup \\ R^1—(OCH_2CH_2)_n \end{array}$$

in which $R^1$ denotes $C_8—C_{26}$-alkyl or $C_9—C_{18}$-alkylaryl, $R^2$ denotes $C_1—C_{22}$-alkyl, and n is a number from 1 to 15, which comprises reacting 2 moles of a compound of the formula

$$R^1—(OCH_2CH_2)_n—X$$

in which X denotes halogen or $—OSO_3Na$ or $—OSO_3K$, and $R^1$ and n are as defined above, with 1 to 1.4 moles of a compound of the formula

$$R^2—NH_2$$

where $R^2$ denotes a $C_1—C_{22}$-alkyl radical, at temperatures of 100 to 200°C, preferably 160 to 185°C, in a solvent or solvent mixture preferably in water, in the presence of an at least equimolar amount of a strong base.